# EUROPÄISCHE PATENTSCHRIFT

(11) **EP 3 223 708 B1**
(45) Veröffentlichungstag und Bekanntmachung des Hinweises auf die Patenterteilung: **24.07.2019**
(21) Anmeldenummer: 15808364.2
(22) Anmeldetag: 25.11.2015
(51) Int. Cl.: A61B 6/14, A61B 6/00

(54) **VERFAHREN FÜR DIE DIAGNOSTIK IN DER KIEFERORTHOPÄDIE**
ORTHODONTIC DIAGNOSTIC METHOD
PROCÉDÉ DIAGNOSTIQUE EN ORTHOPÉDIE MAXILLAIRE

(30) Priorität: 25.11.2014 DE 102014223967
(43) Veröffentlichungstag der Anmeldung: 04.10.2017
(73) Patentinhaber: Sirona Dental Systems GmbH, 64625 Bensheim (DE)
(72) Erfinder: ABKAI, Ciamak, 68542 Heddesheim (DE); LINDENBERG, Kai, 64395 Wersau (DE); LUDWIG, Björn, 56841 Traben-Trarbach (DE)
(74) Vertreter: Sommer, Peter
(86) Internationale Anmeldenummer: PCT/EP2015/077594
(87) Internationale Veröffentlichungsnummer: WO 2016/083431

(56) Entgegenhaltungen:
- DE-A1-102012 221 374
- US-A1- 2003 169 913
- US-A1- 2007 258 638
- US-A1- 2011 268 327
- US-A1- 2012 063 564

## Beschreibung

### Technisches Gebiet

Die Erfindung betrifft ein Verfahren für die Diagnostik in der Kieferorthopädie, wobei mindestens eine initiale zweidimensionale Röntgenaufnahme eines Kopfes aufgenommen wird.

### Stand der Technik

Aus dem Stand der Technik sind mehrere Verfahren zur Diagnostik in der Kieferorthopädie bekannt.

US 2011/268327 A1 offenbart ein Verfahren und eine Vorrichtung für einen Kieferorthopäden, wobei ein integriertes dreidimensionales Modell eines Gebisses und der umgebenden Anatomie eines Patienten erzeugt wird. Das dreidimensionale Modell wird aus dreidimensionalen optischen Bilddaten, die mittels eines optischen intraoralen Scanners erzeugt werden, und aus dreidimensionalen Röntgendaten, die mittels einer CRT-Vorrichtung erzeugt werden, registriert. Zur Erzeugung des Patientenmodells können auch Farbfotographien des Gesichtes und des Kopfes des Patienten verwendet werden. Zur Verlaufsdiagnostik einer Behandlung kann das dreidimensionale Patientenmodell periodisch in mehreren Zeitabständen erfasst werden.

DE 10 2012 221 374 A1 offenbart ein Verfahren zur Planung einer dentalen Behandlung, wobei mittels mindestens einer Videokamera eine Videoaufnahme aufgenommen wird, wobei ein Patientenmodell vorhanden ist, das Bilddaten des Objekts umfasst, wobei die Videoaufnahme an das Patientenmodell so gekoppelt ist, dass beim Bewegen der Videokamera relativ zum Objekt eine Blickrichtung der Ansicht auf das Patientenmodell abhängig von einer sich ändernden Aufnahmerichtung der Videoaufnahme verändert wird. Das Patientenmodell kann dabei dreidimensionale Röntgendaten umfassen.

US 2007/258638 A1 offenbart ein Verfahren zur Diagnose einer Zahnsituation, wobei ein Patientenmodell aus dreidimensionalen Röntgendaten erzeugt wird.

US 2003/169913 A1 offenbart ein Verfahren zur Bildbearbeitung in der Zahnmedizin, wobei ein zweidimensionales Bild und ein dreidimensionales Bild kombiniert werden, um die Informationen beider Bilder zu erhalten. Bei der Kombination der beiden Bilder werden bestimmte Markierungen definiert, um die Registrierung der beiden Bilder durchzuführen. Das dreidimensionale Bild kann mittels eines MRI-Verfahrens oder eines CRT-Verfahrens aufgenommen werden. Die Markierungen in den beiden Aufnahmen können typische Strukturen der Zähne sein, beispielsweise wie der Schneidezähne oder der Backenzähne. Ein virtuelles Modell kann verwendet werden, um mögliche Verschiebungen der Zähne zu simulieren.

US 2012/0063564 A1 offenbart ein Verfahren zur Registrierung von dreidimensionalen Röntgendaten mit einem zweidimensionalen Projektionsbild, wobei eine Projektionsrichtung festgelegt wird und eine Projektion simuliert wird. Anschließend werden die simulierten Röntgendaten mit den zweidimensionalen Röntgendaten in Überlagerung gebracht. Bei einer cephalometrischen Analyse wird eine seitliche cephalometrische Röntgenaufnahme durchgeführt und charakteristische Strukturen in dieser cephalometrische Röntgenaufnahme vermessen. Dabei werden unterschiedliche kieferorthopädische Messgrößen ermittelt, wie beispielsweise eine Frankfurter Horizontale, ein Abstand zwischen dem Kinn und dem Kiefergelenk oder ein Abstand zwischen der Schädelbasis und dem Kiefergelenk.

Ein Nachteil dieses Verfahrens besteht darin, dass die kieferorthopädischen Messgrößen bei hohem Zeitaufwand manuell in der seitlichen cephalometrischen Röntgenaufnahme ausgemessen werden.

Bei einer Verlaufsdiagnostik werden in regelmäßigen Zeitabständen mehrere cephalometrische Röntgenaufnahmen durchgeführt, wobei in jeder der zweidimensionalen cephalometrischen Röntgenaufnahmen die wesentlichen kieferorthopädischen Messgrößen ausgemessen werden und die zeitabhängige Veränderung dieser Messgrößen abhängig von der Behandlung beurteilt werden kann.

Ein weiterer Nachteil der klassischen cephalometrischen Analyse besteht darin, dass die ausgemessenen kieferorthopädischen Messgrößen abhängig von der Positionierung und der Ausrichtung des Patientenkopfes sind. Eine fehlerhafte Positionierung des Patientenkopfes kann also zu Messfehlern führen.

Die Aufgabe der völligen Erfindung besteht also darin, ein Verfahren zur Diagnostik in der Kieferorthopädie bereit zu stellen, das eine sichere, fehlerfreie und Zeit sparende Analyse und Ermittlung der kieferorthopädischen Messgrößen ermöglicht.

### Darstellung der Erfindung

Die Erfindung betrifft ein Verfahren für die Diagnostik in der Kieferorthopädie, wobei mindestens eine initiale zweidimensionale Röntgenaufnahme eines ersten Bereichs eines Kopfes aufgenommen wird. Anschließend wird mindestens eine dreidimensionale Röntgenaufnahme eines zweiten Bereichs einer Zahnsituation aufgenommen, wobei die dreidimensionale Röntgenaufnahme mit der initialen zweidimensionalen Röntgenaufnahme mittels eines Registrierungsverfahrens zu einer Gesamtaufnahme kombiniert wird, wobei zur Verlaufsdiagnostik nacheinander in regelmäßigen zeitlichen Abständen mehrere dreidimensionale Röntgenaufnahmen des zweiten Bereichs der Zahnsituation aufgenommen werden, wobei der erste Bereich des Kopfes den zweiten Bereich (5) der Zahnsituation umfasst, wobei der erste Bereich des Kopfes bestimmte Fixpunkte, wie einen Schädelbasispunkt oder einen Kiefergelenk, enthält, wobei die initiale zweidimensionale Röntgenaufnahme eine seitliche cephalometrische Aufnahme ist.

Die seitliche cephalometrische Aufnahme enthält also den größeren ersten Bereich, der die Schädelbasis, das Kiefergelenk, das Kinn, den Oberkiefer und den Unterkiefer umfasst.

Die initiale zweidimensionale Röntgenaufnahme kann eine seitliche cephalometrische zweidimensionale Röntgenaufnahme sein, die zumindest einen Teilbereich des Kopfes eines Patienten aufnimmt. Der aufgenommene erste Bereich kann beispielsweise charakteristische Fixpunkte, wie das Kiefergelenk oder charakteristische Punkte der Schädelbasis enthalten. Die bekannten Fixpunkte können dann für eine komparative Überlagerung bei der Registrierung verwendet werden. Die dreidimensionale Röntgenaufnahme kann eine DVT-Aufnahme sein, die den zweiten Bereich aufnimmt, der beispielsweise den Unterkiefer umfasst. Es können zusätzlich zu der ersten dreidimensionalen Röntgenaufnahme auch weitere dreidimensionale Röntgenaufnahmen in regelmäßigen zeitlichen Abständen aufgenommen werden, um eine Verlaufsdiagnostik durchzuführen. Das Registrierungsverfahren kann mittels eines Computers automatisch erfolgen, wobei die zweidimensionale Röntgenaufnahme mit der dreidimensionalen Röntgenaufnahme kombiniert wird. Dabei können charakteristische Strukturen in den beiden Aufnahmen, wie eine charakteristische Form des Unterkiefers, die Form des Oberkiefers, das Kiefergelenk oder das Kinn, verwendet werden. Beim Registrierungsverfahren kann ein Optimierungsalgorithmus verwendet werden, der die Methode der kleinsten Quadrate anwendet, wobei die zu optimierenden in der Regel transitorischen und rotatorischen Parameter schrittweise verändert werden, um zu einer optimalen Lösung zu gelangen. Zur Beschleunigung dieses Optimierungsalgorithmus kann der Suchraum verkleinert werden, wobei Informationen über die Positionierung des Kopfes relativ zum verwendeten Röntgengerät berücksichtigt werden.

Ein Vorteil dieses Verfahrens besteht darin, dass zur Diagnostik lediglich ein kleiner zweiter Bereich der Zahnsituation aufgenommen wird, wobei die dreidimensionale Röntgenaufnahme mit der initialen zweidimensionalen Röntgenaufnahme eines deutlich größeren Bereichs des Kopfes registriert wird. Bei einer Reihe von dreidimensionalen Röntgenaufnahmen wird jede der dreidimensionalen Röntgenaufnahmen mit derselben initialen zweidimensionalen Röntgenaufnahme registriert. Dadurch wird also im Vergleich zu einer Reihe von dreidimensionalen Röntgenaufnahmen des gesamten Kopfes die Gesamtdosis minimiert.

Ein weiterer Vorteil des Verfahrens besteht darin, dass im Vergleich zu der klassischen cephalometrischen Analyse eine fehlerhafte Positionierung des Patienten unproblematisch ist, da die Abstände in der dreidimensionalen Röntgenaufnahme zwischen relevanten Messpunkten im Vergleich zu einer projizierten zweidimensionalen Aufnahme unverändert bleiben. Denn bei einer leichten Drehung des Patientenkopfes werden die Projektion des Patientenkopfes auf der zweidimensionalen Röntgenaufnahme und damit auch die Abstände zwischen den relevanten Messpunkte verändert.

Ein weiterer Vorteil dieses Verfahrens besteht darin, dass die kieferorthopädischen Messgrößen anhand der kombinierten Gesamtaufnahme mittels eines Computers automatisch ermittelt werden können. Im Vergleich zum manuellen Ausmessen bei der klassischen cephalometrischen Analyse wird also der Zeitaufwand vermindert und mögliche Fehler beim manuellen Ausmessen verhindert.

Vorteilhafterweise können nacheinander in regelmäßigen zeitlichen Abständen mehrere dreidimensionale Röntgenaufnahmen des zweiten Bereichs der Zahnsituation aufgenommen werden.

Dadurch wird also eine Verlaufsdiagnostik in Abhängigkeit von der Zeit ermöglicht. Bei bestimmten kieferorthopädischen Behandlungen kann dadurch also der Heilungsprozess oder der Verlauf einer Zahnkorrektur mittels einer Zahnspange überprüft werden.

Vorteilhafterweise kann die dreidimensionale Röntgenaufnahme eine DVT-Aufnahme sein.

Die DVT-Aufnahme enthält also einen kleineren Bereich, der beispielsweise den Unterkiefer, den Oberkiefer oder auch eine Gruppe von Zähnen umfasst.

Vorteilhafterweise kann der zweite Bereich einen Oberkiefers und/oder einen Unterkiefers umfassen.

Dadurch wird also die gesamte Zahnsituation vermessen.

Vorteilhafterweise kann der erste Bereich des Kopfes den zweiten Bereich der Zahnsituation umfassen, wobei der erste Bereich des Kopfes bestimmte Fixpunkte, wie einen Schädelbasispunkt oder einen Kiefergelenk, enthält.

Die Fixpunkte sind also anatomische stabile Strukturen, die bei einer kieferorthopädischen Behandlung unverändert relativ zum Schädel bleiben und dadurch als Bezugspunkte beim Ausmessen bestimmter charakteristischer Abstände beziehungsweise kieferorthopädischer Messgrößen dienen können.

Vorteilhafterweise kann mindestens eine orthopädische Messgröße eines Kiefers, eines Zahns und/oder mehrerer Zähne relativ zu einem Fixpunkt ermittelt werden.

Dadurch kann also bei einer Zahnkorrektur die zeitliche Lageänderung eines einzelnen Zahns oder mehrerer Zähne relativ zum Schädel ermittelt und dargestellt werden. Diese Verlaufsdiagnostik ermöglicht dann dem behandelnden Arzt die kieferorthopädische Behandlung anzupassen.

Vorteilhafterweise kann zur Ermittlung der orthopädischen Messgröße anhand der kombinierten Gesamtaufnahme eine Segmentierung und/oder eine Klassifizierung von kieferorthopädischen Strukturen, wie eines Oberkiefers, eines Unterkiefers, einer Schädelbasis, ein Gaumendach oder eines Kiefergelenks, mittels eines Computers automatisch durchgeführt werden.

Durch die Segmentierung werden also die genannten Strukturen automatisch gegenüber ihrem Umgebungsgewebe abgegrenzt und anschließend anhand ihrer charakteristischen Form klassifiziert. Die segmentierten charakteristischen Strukturen können in der grafischen Darstellung auch farblich hervorgehoben werden.

Die segmentierten kieferorthopädischen Strukturen, die sowohl in der zweidimensionalen Röntgenaufnahme als auch in der dreidimensionalen Röntgenaufnahme vorhanden sind, können zur Registrierung verwendet werden, indem diese Strukturen in beiden zu registrierenden Röntgenaufnahmen überlagert werden.

Die Segmentierung kann auch teilweise automatisch erfolgen, wobei der Benutzer manuell einen Punkt oder mehrere Punkte auf einer Grenze der Struktur auswählt und anschließend unter Berücksichtigung der ausgewählten Punkte die vollständige Segmentierung dieser Struktur automatisch mittels des Computers erfolgt.

Vorteilhafterweise kann ein zeitlicher Verlauf einer segmentieren kieferorthopädischen Struktur anhand der nacheinander aufgenommenen dreidimensionalen Röntgenaufnahmen ermittelt werden.

Dadurch kann also eine Verlaufsdiagnostik für eine bestimmte kieferorthopädische Struktur, wie den Oberkiefer, den Unterkiefer, ein einzelnen Zahn oder auch eine Gruppe von Zähnen durchgeführt werden.

Vorteilhafterweise kann zur Verlaufsdiagnostik der Verlauf der kieferorthopädischen Struktur in Abhängigkeit von der Zeit graphisch dargestellt werden.

Dadurch ermöglicht die grafische Darstellung der Verlaufsdiagnostik dem behandelnden Arzt den Verlauf der Behandlung zu beurteilen und die Behandlung gegebenenfalls anzupassen.

Vorteilhafterweise kann die Registrierung zwischen der initialen zweidimensionalen Röntgenaufnahme und der mindestens einen dreidimensionalen Röntgenaufnahme unter Verwendung bekannter Geometriemaße eines verwendeten Röntgengeräts durchgeführt werden, wobei eine perspektivische Verzerrung eines aufgenommenen Objekts in der zweidimensionalen Röntgenaufnahme berücksichtigt wird.

Bei der seitlichen cephalometrischen Aufnahme kann die perspektivische Verzerrung anhand der bekannten geometrischen Größen des Röntgengeräts ermittelt werden. Diese Größen sind der Abstand zwischen dem Sensor und dem Objekt, wie dem Patientenkopf, der Abstand zwischen Objekt und der Röntgenquelle, die Einstellung der Aktuatoren und die Einstellung der Blenden. Bei der Registrierung kann ein Optimierungsalgorithmus angewendet werden, wobei bekannte Toleranzen der mechanischen Elemente, wie der Aktuatoren und der Blenden, den Parameterraum eines Suchraumes stark einschränken.

Vorteilhafterweise kann bei der Registrierung zwischen der initialen zweidimensionalen Röntgenaufnahme und der mindestens einen dreidimensionalen Röntgenaufnahme eine bekannte Position und/oder Ausrichtung eines aufzunehmenden Objekts, wie eines Unterkiefer und/oder eines Oberkiefer, relativ zu einem Röntgengerät verwendet werden.

Die Positionierung des Patienten und damit des aufzunehmenden Objekts wird also bei der Registrierung berücksichtigt. Die Position und die Ausrichtung des Patientenkopfes können mittels bestimmter Positionierungsmittel, wie einer Stirnstütze und einer Kinnauflage, am Röntgengerät eingestellt werden. Die Informationen über die Position und die Ausrichtung des Patientenkopfes verkleinert also deutlich den Lösungsraum bei der Anwendung des Optimierungsalgorithmus.

Vorteilhafterweise kann die bekannte Position und/oder die bekannte Ausrichtung des aufzunehmenden Objekts relativ zum Röntgengerät beim Initiieren eines Suchraumes und/oder einer Startlösung bei einem Optimierungsalgorithmus berücksichtigt werden.

Dadurch wird also die Rechenzeit für die Registrierung vermindert und mögliche Registrierungsfehler verhindert. Eine Startlösung für den Optimierungsalgorithmus kann also auf wenige Zentimeter genau im Vergleich zur tatsächlichen Position und Ausrichtung des Patientenkopfes gewählt werden. Der verkleinerte Suchraum führt dazu, dass das Risiko vermindert wird nach dem Durchlauf des automatisierten Optimierungsalgorithmus im falschen lokalen Minimum zu landen.

### Kurzbeschreibung der Zeichnungen

Die Erfindung wird anhand der Zeichnungen erläutert. Es zeigt, die
- Fig. 1: eine Skizze zur Verdeutlichung des vorliegenden Verfahrens mit einer kombinierten Gesamtaufnahme; die
- Fig. 2: eine Skizze eines zeitabhängigen Verlaufs der Zähne und der Zahnwurzel bei einer Zahnkorrektur; die
- Fig. 3: eine Skizze eines zeitabhängigen Verlaufs der Zähne und der Zahnwurzel bei einer alternativen Zahnkorrektur.

### Ausführungsbeispiele

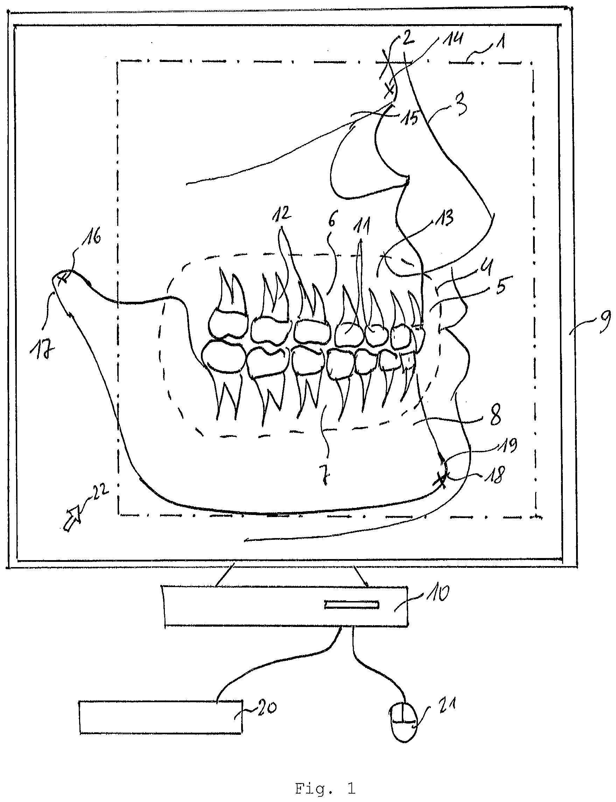

Die Fig. 1 zeigt eine Skizze zur Verdeutlichung des vorliegenden Verfahrens der Diagnostik in der Kieferorthopädie. Im ersten Verfahrensschritt wird eine initiale zweidimensionale Röntgenaufnahme 1 eines ersten Bereichs 2 aufgenommen, wobei der erste Bereich 2 zumindest teilweise einen Kopf 3 eines Patienten umfasst. Die zweidimensionale Röntgenaufnahme ist im vorliegenden Fall eine seitliche cephalometrische Röntgenaufnahme. Anschließend wird mindestens eine dreidimensionale Röntgenaufnahme 4 eines zweiten Bereichs 5, der gestrichelt dargestellt ist, aufgenommen, wobei der zweite Bereich 5 in folgendem Fall einen Oberkiefer 6 und einen Unterkiefer 7 umfasst. Die Begrenzung des ersten Bereichs 2 ist durch eine Strichpunktlinie dargestellt. Im weiteren Verfahrensschritt wird die zweidimensionale Röntgenaufnahme 1 mit der dreidimensionalen Röntgenaufnahme 4 unter Verwendung eines Registrierungsverfahrens zu einer Gesamtaufnahme 8 kombiniert. Die kombinierte Gesamtaufnahme 8 wird mittels einer Anzeigevorrichtung 9, wie eines Monitors grafisch dargestellt. Das Kombinieren der initialen zweidimensionalen Röntgenaufnahme 1 und der dreidimensionalen Röntgenaufnahme 4 erfolgt mittels eines Computers 10, wobei ein Optimierungsalgorithmus angewendet wird. Dabei werden charakteristische Strukturen, die einzelne Zähne 11, Zahnwurzeln 12 oder charakteristische Verläufe eines Kieferknochens 13 in den beiden Röntgenaufnahmen 1 und 4 in Überlagerung gebracht. Der Optimierungsalgorithmus kann beispielsweise auf ein Verfahren der kleinsten Quadrate beruhen.

Zusätzlich zu der ersten dreidimensionalen Röntgenaufnahme 4 können weitere dreidimensionale Röntgenaufnahmen des Bereichs 5 in regelmäßigen zeitlichen Abständen aufgenommen werden, sodass eine Verlaufsdiagnostik ermöglicht wird. Dabei können beispielsweise die Änderungen der Lage und der Ausrichtung der Zähne 11, sowie der Zahnwurzel 12 bei einer Zahnkorrekturbehandlung mittels einer Zahnspange durchgeführt werden. Der erste Bereich 2 kann bestimmte Fixpunkte enthalten, die einen ersten Fixpunkt 14 an der Stirnseite der Schädelbasis 15 und einen zweiten Fixpunkt 16 am Kiefergelenk 17. Anhand der kombinierten Gesamtaufnahme 8 können bestimmte kieferorthopädische Abstände ermittelt werden, wie beispielsweise ein Abstand zwischen dem zweiten Fixpunkt 16 am Kiefergelenk 17 und einer Spitze 18 des Kinns 19. Anhand der Gesamtaufnahme 8 kann auch die Lage und die Ausrichtung der einzelnen Zähne 11 und der Zahnwurzeln 12 relativ zu den Fixpunkten 14 und 16 ermittelt werden. An dem Computer 10 sind herkömmliche Bedienungsmittel, wie eine Tastatur 20 und eine Maus 21 angeschlossen, sodass der Benutzer mittels eines Cursors 22 bestimmte Bereiche oder Strukturen in der Gesamtaufnahme 8 auswählen kann. Die ausgewählte Struktur kann farblich hervorgehoben werden. Die Ermittlung der kieferorthopädischen Abstände beziehungsweise Messgrößen kann mittels des Computers 10 automatisch erfolgen, wobei eine Segmentierung und/oder eine Klassifizierung der kieferorthopädischen Strukturen, wie der Oberkiefer 6, der Unterkiefer 7, die Schädelbasis 15 oder das Kiefergelenk 17 automatisch erfolgen kann. Das Ergebnis des automatischen Verfahrens kann also eine Tabelle mit den wesentlichen kieferorthopädischen Messgrößen sein.

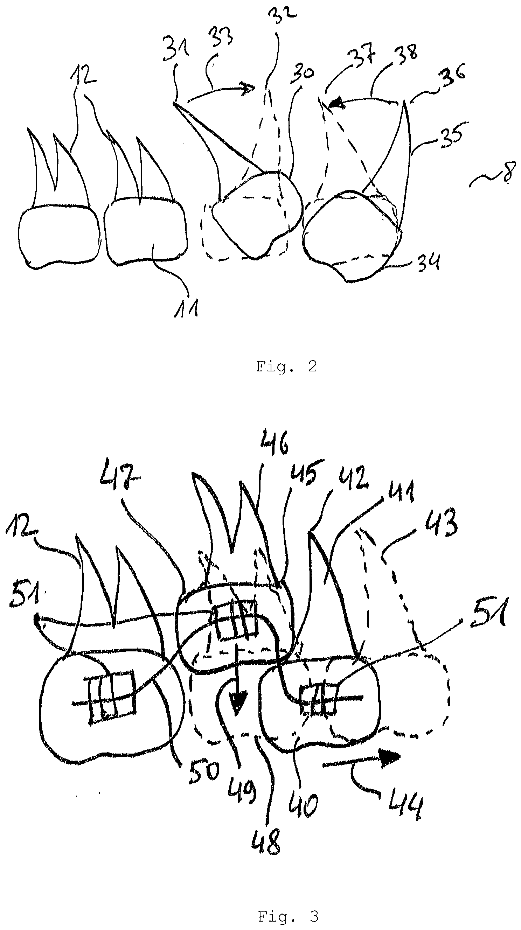

Die Fig. 2 zeigt eine Skizze der Zähne 11 und der Zahnwurzel 12 bei einer Zahnkorrektur. Ein erster Zahn 30 mit einer ersten Zahnwurzel 31 wird im Verlauf der Zahnkorrekturbehandlung aus einer Anfangsposition in eine Endposition 32 gebracht, die gestrichelt dargestellt ist, wobei die Lageänderung durch den Pfeil 33 angedeutet ist. Ein zweiter Zahn 34 mit einer zweiten Zahnwurzel 35 wird aus einer Anfangsposition 36 in eine Endposition 37, die gestrichelt dargestellt ist, am Ende der Zahnkorrekturbehandlung gebracht, wobei die Lageänderung durch einen Pfeil 38 angedeutet ist. Der zweite Zahn 34 und die zweite Zahnwurzel ist also in der Anfangsposition 36 in einer ersten dreidimensionalen Röntgenaufnahme einer Aufnahmereihe am Anfang der Zahnkorrekturbehandlung und der Endposition 37 in einer letzten dreidimensionalen Aufnahme am Ende dieser Aufnahmereihe abgebildet. Der Verlauf der Lageänderung lässt sich also grafisch in Abhängigkeit von der Zeit mitverfolgen.

Die Fig. 3 zeigt eine Skizze der Zähne 11 und der Zahnwurzel 12, wobei ein dritter Zahn 40 mit einer dritten Zahnwurzel 41 aus einer Anfangsposition 42, die durch eine volle Linie dargestellt ist, in eine Endposition 43, die durch eine gestrichelte Linie dargestellt ist, während der Zahnkorrekturbehandlung mittels einer Zahnspange gebracht ist, wobei die Lageänderung durch den Pfeil 44 angedeutet ist. Darüber hinaus wird ein vierter Zahn 45 mit einer vierten Zahnwurzel 46 aus einer Anfangsposition 47 in eine Endposition 48 nach der Zahnkorrektur bewegt, die gestrichelt dargestellt ist. Die Lageänderung des vierten Zahns 45 ist dabei durch den Pfeil 49 angedeutet. Die Lageänderung bzw. Korrektur der einzelnen Zähne 11, 40 und 45 kann dabei mittels einer Zahnspange 50 mit den an den Zähnen angebrachten Brackets 51 erfolgen.

### Bezugszeichen

- 1: Initiale zweidimensionale Röntgenaufnahme
- 2: Erster Bereich
- 3: Kopf
- 4: Dreidimensionale Röntgenaufnahme
- 5: Zweiter Bereich
- 6: Oberkiefer
- 7: Unterkiefer
- 8: Gesamtaufnahme
- 9: Anzeigevorrichtung
- 10: Computer
- 11: Zähne
- 12: Zahnwurzel
- 13: Kieferknochen
- 14: Erster Fixpunkt
- 15: Schädelbasis
- 16: Zweiter Fixpunkt
- 17: Kiefergelenk
- 18: Spitze
- 19: Kinn
- 20: Tastatur
- 21: Maus
- 22: Cursor
- 30: Erster Zahn
- 31: Erste Zahnwurzel
- 32: Endposition
- 33: Pfeil
- 34: Zweiter Zahn
- 35: Zweite Zahnwurzel
- 36: Anfangsposition
- 37: Endposition
- 38: Pfeil
- 40: dritter Zahn
- 42: Anfangsposition
- 43: Endposition
- 44: Pfeil
- 45: vierter Zahn
- 46: vierte Zahnwurzel
- 47: Anfangsposition
- 48: Enposition
- 49: Lageänderung
- 50: Zahnspange
- 51: Brackets

## Patentansprüche

1. Verfahren für die Diagnostik in der Kieferorthopädie, wobei mindestens eine initiale zweidimensionale Röntgenaufnahme (1) eines ersten Bereichs (2) eines Kopfes (3) aufgenommen wird, wobei anschließend mindestens eine dreidimensionale Röntgenaufnahme (4) eines zweiten Bereichs (5) einer Zahnsituation aufgenommen wird, wobei die dreidimensionale Röntgenaufnahme (4) mit der initialen zweidimensionalen Röntgenaufnahme (1) mittels eines Registrierungsverfahrens zu einer Gesamtaufnahme (8) kombiniert wird, wobei zur Verlaufsdiagnostik nacheinander in regelmäßigen zeitlichen Abständen mehrere dreidimensionale Röntgenaufnahmen (4) des zweiten Bereichs (5) der Zahnsituation aufgenommen werden, wobei der erste Bereich (2) des Kopfes (3) den zweiten Bereich (5) der Zahnsituation umfasst, wobei der erste Bereich (2) des Kopfes (3) bestimmte Fixpunkte (14, 16), wie einen Schädelbasispunkt oder einen Kiefergelenk, enthält, wobei die initiale zweidimensionale Röntgenaufnahme (1) eine seitliche cephalometrische Aufnahme ist.

2. Verfahren nach Anspruch 1, **dadurch gekennzeichnet, dass** die dreidimensionale Röntgenaufnahme (4) eine DVT-Aufnahme ist.

3. Verfahren nach Anspruch 1 oder 2, **dadurch gekennzeichnet, dass** der zweite Bereich (5) einen Oberkiefer und/oder einen Unterkiefer (7) umfasst.

4. Verfahren nach einem der Ansprüche 1 bis 3, **dadurch gekennzeichnet, dass**, zur Diagnostik mindestens eine orthopädische Messgröße eines Kiefers, eines Zahns und/oder mehrerer Zähne (11) relativ zu einem Fixpunkt (14) ermittelt wird.

5. Verfahren nach Anspruch 4, **dadurch gekennzeichnet, dass** zur Ermittlung der orthopädischen Messgröße anhand der kombinieren Gesamtaufnahme (8) eine Segmentierung und/oder eine Klassifizierung von kieferorthopädischen Strukturen, wie eines Oberkiefers (6), eines Unterkiefers (7), einer Schädelbasis (15) oder eines Kiefergelenks, mittels eines Computers (10) automatisch durchgeführt wird.

6. Verfahren nach Anspruch 5, **dadurch gekennzeichnet, dass** ein zeitlicher Verlauf (33, 38, 44) einer segmentieren kieferorthopädischen Struktur anhand der nacheinander aufgenommenen dreidimensionalen Röntgenaufnahmen (4) ermittelt wird.

7. Verfahren nach Anspruch 6, **dadurch gekennzeichnet, dass** zur Verlaufsdiagnostik der Verlauf der kieferorthopädischen Struktur in Abhängigkeit von der Zeit graphisch dargestellt wird.

8. Verfahren nach einem der Ansprüche 1 bis 7, **dadurch kennzeichnet, dass** die Registrierung zwischen der initialen zweidimensionalen Röntgenaufnahme (1) und der mindestens einen dreidimensionalen Röntgenaufnahme (4) unter Verwendung bekannter Geometriemaße eines verwendeten Röntgengeräts durchgeführt wird, wobei eine perspektivische Verzerrung eines aufgenommenen Objekts in der zweidimensionalen Röntgenaufnahme (1) berücksichtigt wird.

9. Verfahren nach einem der Ansprüche 1 bis 8, **dadurch gekennzeichnet, dass** bei der Registrierung zwischen der initialen zweidimensionalen Röntgenaufnahme (1) und der mindestens einen dreidimensionalen Röntgenaufnahme (4) eine bekannte Position und/oder Ausrichtung eines aufzunehmenden Objekts, wie eines Unterkiefers (7) und/oder eines Oberkiefers (6), relativ zu einem aufzunehmenden Röntgengerät verwendet wird.

10. Verfahren nach Anspruch 9, **dadurch gekennzeichnet, dass** die bekannte Position und/oder Ausrichtung des aufzunehmenden Objekts relativ zum Röntgengerät beim Initiieren eines Suchraumes und/oder einer Startlösung bei einem Optimierungsalgorithmus berücksichtigt werden.

## Claims

1. Method for diagnostics in orthodontics, wherein at least one initial two-dimensional X-ray image (1) of a first region (2) of a head (3) is recorded, wherein subsequently at least one three-dimensional X-ray image (4) of a second region (5) of a tooth situation is recorded, wherein the three-dimensional X-ray image (4) is combined with the initial two-dimensional X-ray image (1) by means of a registration process to form an overall image (8), wherein a plurality of three-dimensional X-ray images (4) of the second region (5) of the tooth situation are successively recorded at regular time intervals for the process diagnostics, wherein the first region (2) of the head (3) comprises the second region (5) of the tooth situation, wherein the first region (2) of the head (3) includes specific fixed points (14, 16), such as a cranial base point or an temporomandibular joint, wherein the initial two-dimensional X-ray image (1) is a lateral cephalometric image.

2. Method according to Claim 1, **characterized in that** the three-dimensional X-ray image (4) is a DVT image.

3. Method according to Claim 1 or 2, **characterized in that** the second region (5) comprises an upper jaw and/or a lower jaw (7).

4. Method according to any one of Claims 1 to 3, **characterized in that** at least one orthopaedic measured value of a jaw, a tooth and/or a plurality of teeth (11) relative to a fixed point (14) is determined for use in the diagnosis.

5. Method according to Claim 4, **characterized in that**, for the determination of the orthopaedic measured value and using the combined overall image (8), a segmentation and/or classification of orthodontic structures, such as an upper jaw (6), a lower jaw (7), a cranial base (15) or a temporomandibular joint, is carried out automatically by means of a computer (10).

6. Method according to Claim 5, **characterized in that** a time profile (33, 38, 44) of a segmented orthodontic structure is established using the successively recorded three-dimensional X-ray images (4).

7. Method according to Claim 6, **characterized in that**, for the process diagnostics, the profile of the orthodontic structure is graphically displayed as a function of the time.

8. Method according to any one of Claims 1 to 7, **characterized in that** the registration between the initial two-dimensional X-ray image (1) and the at least one three-dimensional X-ray image (4) is carried out using known geometric dimensions of a used X-ray device, wherein a perspective distortion of a recorded object in the two-dimensional X-ray image (1) is taken into account.

9. Method according to any one of Claims 1 to 8, **characterized in that** a known position and/or orientation of an object to be recorded, such as a lower jaw (7) and/or an upper jaw (6), relative to an X-ray device to be used for the recording, is used for the registration between the initial two-dimensional X-ray image (1) and the at least one three-dimensional X-ray image (4).

10. Method according to Claim 9, **characterized in that**, when initiating a search space and/or a starting solution, the known position and/or orientation of the object to be recorded relative to the X-ray device is taken into account in an optimization algorithm.

## Revendications

1. Procédé de diagnostic en orthodontie, dans lequel au moins une radiographie bidimensionnelle initiale (1) d'une première région (2) de la tête (3) est effectuée, où ensuite au moins une radiographie tridimensionnelle (4) d'une deuxième région (5) d'une situation des dents est effectuée, où la radiographie tridimensionnelle (4) est combinée avec la radiographie bidimensionnelle initiale (1) au moyen d'un procédé d'enregistrement pour en faire une radiographie globale (8), où pour le suivi du diagnostic, plusieurs radiographies tridimensionnelles (4) les unes après les autres à intervalles de temps réguliers de la deuxième région (5) de la situation dentaire sont effectuées, où la première région (2) de la tête (3) comprend la deuxième région (5) de la situation dentaire, où la première région (2) de la tête (3) contient des points fixes (14, 16) précis, comme un point de base du crane ou une articulation de maxillaire, où la radiographie bidimensionnelle (1) est une radiographie céphalométrique latérale.

2. Procédé selon la revendication 1, **caractérisé en ce que** la radiographie tridimensionnelle (4) est une tomographie volumétrique par faisceau conique.

3. Procédé selon la revendication 1 ou la revendication 2, **caractérisé en ce que** la deuxième région (5) comprend un maxillaire supérieur et/ou un maxillaire inférieur (7).

4. Procédé selon l'une des revendications 1 à 3, **caractérisé en ce que**, pour le diagnostic, on détermine au moins une mesure orthodontique d'un maxillaire, d'une dent et/ou de plusieurs dents (11) par rapport à un point fixe (14).

5. Procédé selon la revendication 4, **caractérisé en ce que**, pour la détermination de mesure orthodontique à l'aide d'une radiographie globale combinée (8) on effectue automatiquement une segmentation et/ou une classification de structures orthodontiques, comme d'un maxillaire supérieur (6), d'un maxillaire inférieur (7), de la base du crane (15) ou de l'articulation des maxillaires au moyen d'un ordinateur (10).

6. Procédé selon la revendication 5, **caractérisé en ce qu'**une évolution dans le temps (33, 38, 44) d'une structure orthodontique segmentée est déterminée à l'aide des radiographies tridimensionnelles (4) effectuées les unes après les autres.

7. Procédé selon la revendication 6, **caractérisé en ce que**, pour le diagnostic de l'évolution, l'évolution de la structure orthodontique est représentée graphiquement en fonction du temps.

8. Procédé selon l'une des revendications 1 à 7, **caractérisé en ce que** l'enregistrement entre les radiographies bi-dimensionnelles initiales (1) et l'au moins une radiographie tridimensionnelle (4) est effectué moyennant l'emploi de dimensions géométriques connues d'un appareil à rayons X employé, où on tient compte d'une diminution de taille due à la perspective d'un objet radiographié dans la radiographie bidimensionnelle (1).

9. Procédé selon l'une des revendications 1 à 8, **caractérisé en ce que**, lors de l'enregistrement entre la radiographie bidimensionnelle initiale (1) et l'au moins une radiographie tridimensionnelle (4) on emploie une position connue et/ou une orientation de l'objet à radiographier, comme celle d'un maxillaire inférieur (7) et/ou d'un maxillaire supérieur (6) par rapport à un appareil de rayons X.

10. Procédé selon la revendication 9, **caractérisé en ce qu'**on tient compte de la position connue et/ou de l'orientation de l'objet à radiographier par rapport à l'appareil de rayons X lors de l'initialisation d'un espace de recherche et/ou recherche de départ dans un algorithme d'optimisation.
